# EUROPEAN PATENT APPLICATION

(11) **EP 1 382 971 A1**
(43) Date of publication of application: **21.01.2004**
(21) Application number: 02077910.4
(22) Date of filing: 17.07.2002
(51) Int. Cl.: G01N 33/68, C07K 14/47, C07K 16/18

(54) **Detection of prion disease**

(71) Applicant: PEPSCAN SYSTEMS B.V., 8219 PH Lelystad (NL); Stichting Sanquin Bloedvoorziening, 1066 CX Amsterdam (NL)
(72) Inventor: van Oers, Josephus Wilhelmus Alphonsus Maria, 1506 TB Zaandam (NL); Hack, Cornelis Erik, 1111 ND Diemen (NL); Roem-Haagsma, Dorina Dirkje Ina, 1731 SW Winkel (NL); Langeveld, Johannes Pieter Maria, 3844 JB Harderwijk (NL); Garssen, Gerrit Jan, 3872 XC Driebergen (NL); Jacobs, Jorg Günther, 8225 LV Lelystad (NL); van Engelenburg, Franciscus Antonius Cornelis, 3555 EE Utrecht (NL)
(74) Representative: Prins, Adrianus Willem

(57) **Abstract**

This invention relates to the field of the detection of prion diseases. The invention provides a binding molecule or antibody specifically reactive with an epitope which is exposed on a part of an aberrant conformer (PrP^{Sc}) of a prion protein after treatment of said conformer with a protease wherein said epitope is not or only partly exposed on a prion protein which has not been treated with a protease.

## Description

This invention relates to the field of the detection of prion diseases. These diseases, also called transmissible spongiform encephalopathies (TSE), include bovine spongiform encephalopathy (BSE) or mad cow disease in cattle, scrapie in sheep, and Creutzfeldt-Jakob Disease (CJD) in man. Prion diseases are infectious diseases, generally transmitted by a conformer of the naturally present prion protein (PrP), though cases may also occur spontaneously or from a genetic predisposition. Diagnosis of TSE typically requires demonstration of this conformer in animal or human brain.

Prion diseases or TSE are a group of fatal non-inflammatory neurodegenerative disorders examplified by BSE in cattle, scrapie in sheep, CJD in man.¹ TSE are characterised by a relatively long incubation period (e.g. >10 years in human), and typically share accumulation of PrP^{Sc} in brain and in some cases also in lymphoid tissues.^{2,3}

The prion protein is an endogenous protein, that under normal conditions is also expressed in the brain, and also in various other tissues. This normal cellular prion protein (PrP^{C}) and PrP^{Sc} are different conformations of the same protein. Moreover PrP^{C} and PrP^{Sc} differ in some physico-chemical and biochemical properties: PrP^{C} is soluble, exist in a non-aggregated form, and is sensitive to degradation by proteases, whereas PrP^{Sc} is insoluble, exist in an aggregated form, and is relatively resistant to degradation by proteases.⁴ It is hypothesised that a direct protein-protein contact between PrP^{Sc} and PrP^{C} generates a conformational change of prP^{C} into PrP^{Sc}. Ultimately PrP^{Sc} forms aggregates and accumulates in brain. At the final stage of the disease neurodegenerative changes occur in the brain, associated with severe neurological dysfunction, and ultimately death.

The PrP gene of various animal species has been cloned and sequenced.⁵ The protein is encoded within a single exon, and consists of a long N-terminal signal sequence followed by about 250 amino acid (AA) residues. The C-terminus contains a sequence of about 22 residues encoding for linker site for a glycosyl-phosphatidylinositol anchor, a property shared with many other membrane proteins. Furthermore, the protein contains one disulfide bridge and two potential Asparagine-glycosylation sites. The sequence of the protein is well conserved among various animal species. Though differences in the secondary structure of PrP^{Sc} and that of PrP^{C} have been identified, the structural basis for the enhanced aggregatibility and proteolytic resistance of PrP^{Sc} as compared to those of PrP^{C} is still not resolved. In addition, the molecular basis for the existence of different strains of PrP^{Sc}, i.e. prions with different incubation times, is lacking, though it is assumed that these strains likely represent PrP^{Sc} with subtle differences in conformation .⁶

Crucial for the diagnosis of TSE is detection of PrP^{Sc} in brain, using e.g. immuno-histochemistry or Western blotting. Detection of PrP^{Sc} in a more easily accessible sample, preferably blood, would also add to the diagnosis of TSE. However there is only very incomplete data available suggesting that PrP^{Sc} is present in blood.^{7,8} For the in vitro diagnostic methods, polyclonal and monoclonal antibodies are usually applied.^{9, 10} For example, monoclonal antibody 3F4 detects PrP from human, hamsters, and felines. Importantly, it does not react with PrP from any other mammalian species and can thus not be used for the diagnosis of BSE or scrapies. Antibody 3F4 is reactive to both native and denatured forms of PrP and recognizes both protease sensitive and protease resistant forms of PrP. Antibodies against prions described in the literature so far, do not discriminate between PrP^{c} and PrP^{SC}, except for one which can only be used for immunoprecipitation and which is therefore not suitable for regular immuno-diagnostics¹¹

Because most antibodies bind indiscriminately to both conformers, the specificity of diagnostic methods is based on differences in resistance to proteinase K (PK) digestion, solubility, or degree of aggregation. The high background of PrP^{C} in general and in blood in particular, is challenging the specificity of all current immuno-diagnostics, especially in situations when PK digestions cannot be well controlled (e.g. plasma samples, because plasma contains high concentrations of various protease inhibitors).¹²

The invention provides the insight that detectable neo-epitopes are exposed on a part of a protease-digested aberrant conformer (PrP^{Sc}) of a prion protein , such as on scrapie-associated prion protein, and provides the preparation of antibodies against these epitopes and use of such antibodies in diagnostic tests. Such use is in particularly advantageous because it allows a better distinction between aberrant and non-aberrant conformers. The invention provides an antibody (herein also defined as a specific binding molecule or (poly)peptide with antibody properties, such as a single-chain antibody fragment or other molecule provided with a specific complementarity-determining region (CDR) or a binding region mimicking such as binding region, allowing binding to a prion protein or part thereof) specifically reactive with a neo-epitope which is exposed on a part of an aberrant conformer (PrP^{Sc}) of a prion protein after treatment of said conformer with a protease wherein said epitope is not or only partly present or exposed on a prion protein which has not been treated with a protease. Such an antibody as provided by the invention is for example characterized by a higher affinity to said part of said conformer PrP^{Sc} than to a non-aberrant cellular prion protein (PrP^{c}), in particular when said PrP^{Sc} has been treated with a protease, such as the commonly used protease K, or with pronase.

The invention describes for example the exposure of neo-epitopes on scrapie-associated prion protein (PrP^{Sc}) after protease digestion, as well as preparation of antibodies against these epitopes. Antibodies, or other proteins, or compounds specifically reacting with neo-epitopes as described can be used for detection of PrP^{Sc} in tissue or biological fluid samples. Hence, this invention provides new inroads for diagnosis of TSE in animal and human prion diseases.

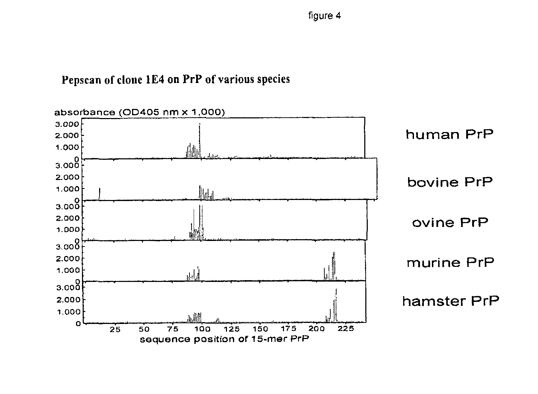

The present invention demonstrates the presence of neo-epitopes on the infectious form of the prion protein, that is PrP^{Sc}, upon proteolytic degradation by PK.
In a preferred embodiment, the invention provides an antibody wherein said epitope comprises a peptide with an amino acid sequence QXXKXS. Such an antibody is in general broadly reactive with aberrant prion proteins from various mammalian species.It is for example preferred that an antibody according to the invention comprises a specific reactivity to overlapping oligo-peptides derived from the human, ovine, murine as well as hamster PrP amino-acid-sequences that correspond to the bovine PrP amino acid sequence at about amino acid position 108-119, as for example determined in Pepscan analyses with overlapping 15-mer oligo-peptides derived from said amino acid sequences as showed herein in the detailed description (Figure 4). Such analyses can however also be done with smaller or longer oligomers, such as for example 11, 12, 13, 14, 16, 17, or 18 -mers. It is preferred that said bovine PrP amino acid sequence comprises GQWNKPSKPKTN. Central to the present invention is the unexpected finding that neo-epitopes are exposed on PrP^{Sc} upon cleavage by protease K. As an example, it is shown that a particular monoclonal antibody (Mab 1E4), raised against a peptide covering the AA sequence GQWNKPSKPKTN of the bovine PrP (AA₁₀₈₋₁₁₉), binds to PK-digested PrP^{Sc} with a high affinity, whereas it has a low affinity for non-digested PrP^{Sc}. The affinity of Mab 1E4 for PrP^{C} is even lower than for non-digested PrP^{Sc}.

Antibodies against these epitopes are exemplified by a monoclonal antibody (Mab) which is derived or derivable from hybridoma cell 1E4 deposited with COLLECTION NATIONALE DE CULTURES DE MICRO-ORGANISMES (CNCM) Institut Pasteur 28, rue du Dr Roux 75724 PARIS Cedex 15 France on July 3 2002 under accession number CNCM I-2906 constitute suitable reagents to detect PrP^{Sc} in biological materials. As antibody, several kinds of binding molecules may be used. For example specific phage-display generated binding peptides are also provided. As a coding sequence for such binding peptides, the invention provides a nucleic acid sequence encoding the amino acid sequence AGDNDAEDY. It is preferred that an antibody according to the invention comprises a V_{H} region encoded by a nucleic acid comprising at least a CDR3 having a nucleic acid sequence encoding the amino acid sequence AGDNDAEDY.

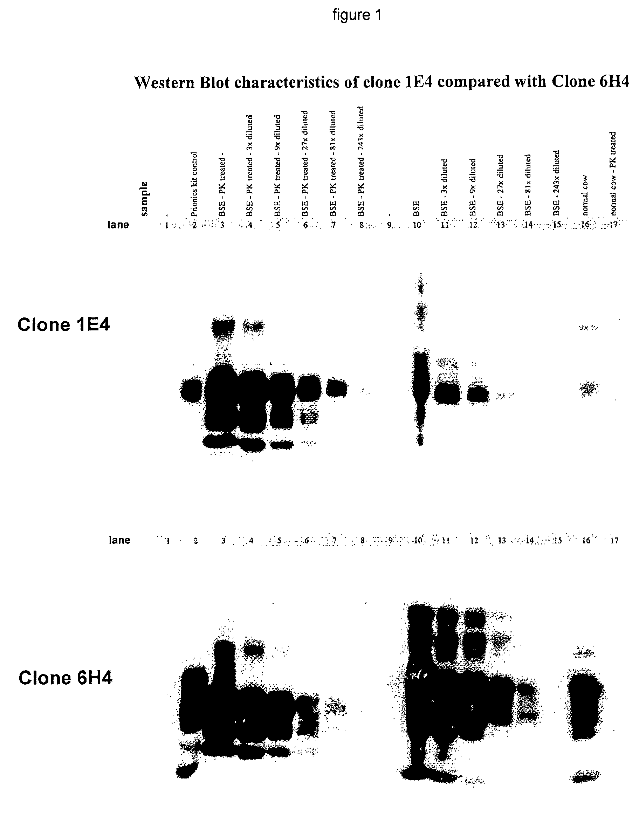

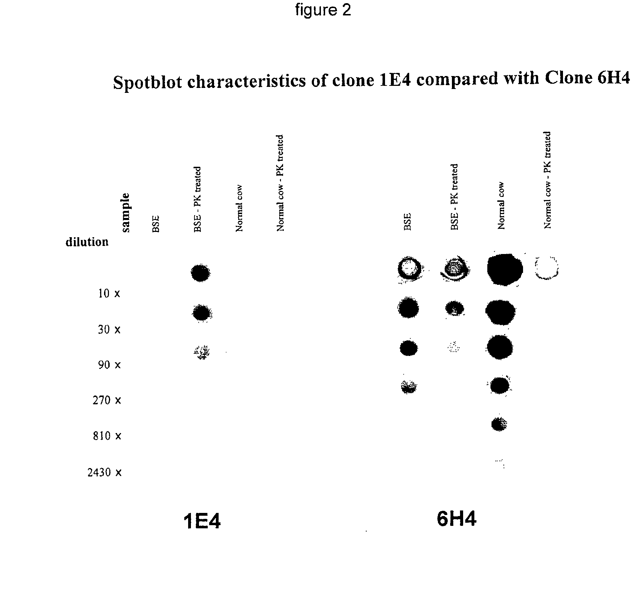

The present invention also provides a method to raise antibodies against said neo-epitopes, and describes the use of these antibodies in assays for PrP^{Sc}.
The invention provides a method for determining the presence of a prion protein in a biological sample via the detection of a complex between said protein and said antibody, said method comprising: contacting a biological sample with an effective amount of antibody according to the invention, under conditions suitable for the formation of said complex, and detecting said complex as indicative of the presence of a prion protein. In a preferred embodiment, the invention provides a method for determining the presence of a prion protein in a biological sample, wherein said method comprises immobilization of proteins present in the sample on a support surface, contacting the sample with a diagnostically effective amount of antibody according to the invention under conditions such that a complex forms between said antibody and said protein and detection of said complex. Furthermore, proteins present in the sample can also be immobilized on various surfaces, such as for example on a polyvinylidene difluoride (PVDF) membrane in a Western-blot analysis or, as another example, on the surface of a microtitre plate in an ELISA-type assay. A suitable method as provided herein (Figure 1) comprises testing for aberrant prions in biological material, for example via an immuno-blotting procedure in which PK-digested prion-containing material is separated, e.g. by sodium dodecyl sulphate polyacrylamide gel-electrophoresis (SDS-PAGE), transferred onto sheets consisting of PVDF or other material, and incubated with a labelled antibody or binding molecule according to the invention that is directed against said prion protein. PrP^{Sc} is discriminated from PrP^{C}, because of its lower apparent molecular weight after PK digestion. Where the antibodies that are in general used for this procedure do not discriminate between PrP^{Sc} and prP^{C} per se, the discrimination is usually made because of the different migration pattern of PK-resistant prion protein on SDS-PAGE. As the present invention shows the existence of neo-epitopes exposed on protease-cleaved prion, antibodies, or other proteins or binding molecules preferentially binding to these neo-epitopes, are superior in detecting PrP^{Sc} since they will not or to a limited extent bind to PrP^{C} present in the sample.
In a preferred embodiment, the type of support surface is a nitrocellulose membrane, as for example shown herein in the detailed description of a spot-blot analysis of BSE-infected brain of cattle using an antibody according to the invention (Figure 2). This analysis shows that said antibody has a low affinity for bovine PrP^{C} and PrP^{Sc}, but a substantially higher affinity for PrP²⁷⁻³⁰ as compared to a more conventional PrP antibody.

In another preferred embodiment, a method is provided for determining the presence of a prion protein in a biological sample comprising at least mixing of said sample in solution with a diagnostically effective amount of antibody according to the invention under conditions such that a complex forms between said antibody and said protein; detecting said complex; and comparing the amount of complex formed with an amount formed when the steps are separately performed using a control sample in place of a biological sample. The invention can for example be used for a direct or a competitive ELISA-type assay.

In yet another preferred embodiment, the invention provides a method for determining the presence of a prion protein in a biological sample comprising at least immobilizing an antibody according to the invention on a support surface, contacting said sample with said antibody under conditions such that a complex forms between said antibody and said protein; and detecting said complex. For example, the antibody can be covalently attached to a solid phase material that can for example be used for chromatographic procedures. Another example is immobilization of said antibody on a sensor chip surface for real time biomolecular interaction analysis (BIA) based on surface plasmon resonance (SPR). Advantageously, the invention provides a method comprising coating the surface of a microtiter plate with an antibody according to the invention, which can then be used for an ELISA-type assay.

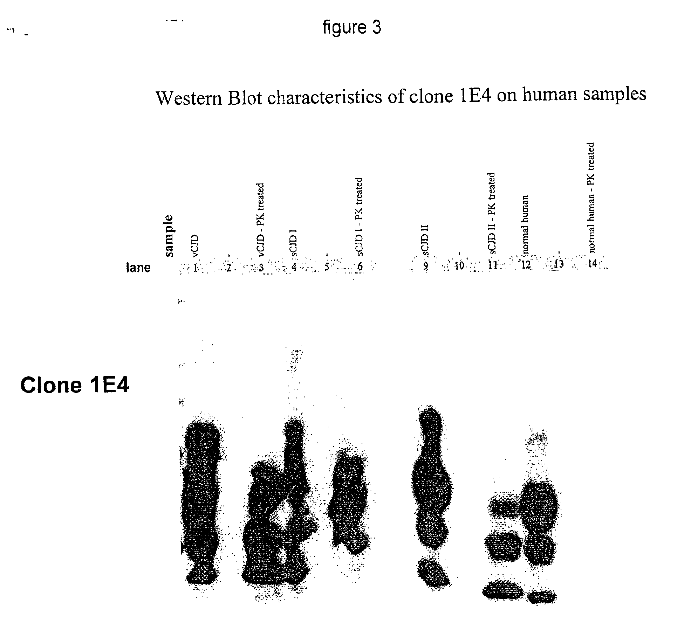

As said, the invention provides a method for determining the presence of a prion protein in a biological sample via the detection of a complex between said protein and an antibody according to the invention. Detection of said complex can be performed by various means. A particular means of detection is the measurement of luminescence, as described herein in the detailed description of a Western blot analysis (Figures 1 and 3) or a spot-blot assay (Figure 2), comprising detection of bound antibodies using a HRP-conjugate and enhanced chemiluminescence (ECL) as substrate. Furthermore, the invention also provides a method wherein said complex is detected by other means such as for example a colorimetric, a fluorescent, a radioactive, an electrical or a magnetic measurement. The invention provides for a diagnostic kit to detect the presence of a prion protein in a biological sample using an antibody according to the invention. As described above, an antibody can be used for various procedures to detect the presence of a prion protein in a biological sample via the detection of a complex between said protein and said antibody, such as for example an ELISA-type assay, a Western blot or an immuno-histochemical assay. In a preferred embodiment, a diagnostic kit contains means for performing a dot-blot assay using an antibody according to the invention to detect the presence of a prion protein in a biological sample.

The present invention also provides a method to raise antibodies against neo-epitopes exposed upon protease-cleavage of PrP^{Sc}. A set of cells of antibody-producing cells is obtained by conventional hybridoma technology known in the field. A mouse is immunised with a prion-protein peptide. Lymphocytes are taken from the spleen and fused to myeloma cells. Hybrid cells are selected and individual hybrid cells are cultured for several days. Aliquots of the culture supernatants are removed and tested for the presence of a monoclonal antibody that reacts with PrP^{Sc} protein via the detection of a complex between said antibody and said protein. Subsequently, an antibody is selected for their ability to react stronger with protease-digested PrP^{Sc} material compared to non-digested PrP^{Sc} to enrich for antibodies against neo-epitopes exposed upon digestion of PrP^{Sc}. Preferably, a selected antibody has at least a six-fold higher affinity for digested PrP^{Sc} than for the non-digested PrP^{Sc}. As shown in Figure 2, a spot-blot analysis can be used to screen for monoclonal antibodies against neo-epitopes exposed upon digestion of PrP^{Sc}, comprising immobilizing serial dilutions of protease-treated or crude brain homogenates derived from normal and infected cattle onto nitrocellulose membrane, drying and blocking said membrane and contacting said membrane with monoclonal antibody supernatants. Bound antibodies can be detected using goat-anti-mouse-immunoglobulin HRP conjugate and ECL as substrate. Clones are selected which produce a monoclonal antibody that is a relatively high affinity for digested PrPSc and a relatively low affinity for non-digested PrPSc and for both the digested and non-digested forms of PrPC. Furthermore, an antibody selection procedure may involve phage display technology which is well known in the field of recombinant antibody production. A host mammal is immunized with PrP protein to mount an immune response. Cells responsible for the production of PrP antibodies are extracted and mRNA is isolated. A cDNA libary is produced by reverse transcription of the RNA. Gene fragments encoding V_{H} and V_{L} are amplified and assembled into a single gene using a DNA linker through PCR. The recombinant DNA fragments can be inserted into a phage display vector such that antibodies are expressed on the phage to obtain a single-chain Fab library. Use of a general panning protocol, involving repeated rounds of sequential binding, washing, elution and reinfection, allows for the selection of antibodies against an antigen. An antigen can be a protein, such as a prion protein. An antigen can also be a synthetic peptide, for example a peptide with an amino acid sequence that corresponds to a prion protein such as PrP^{Sc}. Preferably, said peptide is an epitope of protease-digested PrP^{Sc} that is not available on non-digested PrP^{Sc}.

Advantageously, a selected antibody is characterized by a low affinity for both the digested and the non-digested forms of the non-aberrant cellular prion protein PrP^{C}. In contrast to prion-protein antibodies described so far, an antibody selected according to the invention is highly suitable for immunodiagnostic purposes because it does not detect the high background of PrP^{c} that is observed in general and in blood in particular. The invention provides a method to generate an antibody with a specific reactivity to the amino acid sequences that correspond to the bovine PrP amino acid sequence GQWNKPSKPKTN comprising immunoscreening by ELISA carried out according to established Pepscan procedures. Furthermore, said antibody is preferably characterized by a second specific interaction site with mouse and/or hamster PrP at the amino acid sequence corresponding to the murine amino acid position 217-222. As shown herein (Figure 4), it is preferred that a selected antibody has a specific reactivity to overlapping 15-mer oligopeptides derived from murine as well as hamster PrP amino acid sequence that correspond to murine PrP amino acid sequence at about amino acid position 217-222 in Pepscan analysis.

In a preferred embodiment, a method according to the invention provides an antibody having a high affinity for PrP of a broad range of species, comprising testing a set of antibodies for reactivity of said antibody with oligomerpeptides derived from the human, bovine, ovine, murine and hamster PrP in Pepscan analysis. Such a broadly reactive antibody is suitable as a general antibody for determining the presence of a prion protein in a biological sample, and can be used for the diagnosis of BSE, scrapie or CJD.

The invention is further described in the detailed description.

### Figure legends

### Figure 1

Western blot analysis of BSE-infected brain of cattle using Mabs 1E4 and 6H4 (Prionics AG) demonstrating a neo-epitope on PK-digested PrP^{Sc}. 10% brain homogenates of BSE infected and normal cattle were treated with and without PK and 3-fold serial dilutions were prepared and loaded to two 12% PAA NuPage gels. After electrophoresis and blotting to polyvinylidene difluoride (PVDF) membrane, one blot was stained with Mab 1E4 (upper panel) and one with 6H4 (lower panel). Lanes 1, 9: empty; lane 2: Prionics kit control (1% brain homogenate of normal cattle); lanes 3-8: 3-fold serial dilution of 10% brain homogenate treated with PK; lanes 10-15: 3-fold serial dilution of 10% brain homogenate without PK treatment; lanes 16 and 17: 10% brain homogenate of normal cow without and with PK treatment.

### Figure 2

Spot-blot analysis of BSE-infected brain of cattle using Mabs 1E4 and 6H4. 10% brain homogenates of BSE-infected and normal cattle were treated with and without PK and 3-fold serial dilutions were prepared in SDS-sample buffer and spotted to NC, one membrane was stained with Mab 1E4 (left panel) and one with 6H4 (right panel).

### Figure 3

Western blot analysis of variant- and sporadic-CJD infected human (vCJD/sCJD). 10% brain homogenates of vCJD and sCJD patient and normal human were treated with and without PK and loaded to a 12% PAA NuPage gel. After electrophoresis and blotting to PVDF membrane, the blot was stained with Mab 1E4. Lanes 1, 3: vCJD; lanes 4, 6: sCJD (type 1); lanes 9, 11: sCJD (type 2); lanes 12 and 14, normal human; lanes 1, 4, 9, and 12: not treated with PK; lanes 3, 6, 11, and 14: treated with PK; lanes 2, 5, 10, and 13: empty.

### Figure 4

Pepscan analysis of Mab 1E4. The reactivity of Mab 1E4 to 15-mer oligo-peptides derived from the human, bovine, ovine, murine and hamster PrP amino-acid (AA)-sequences was measured. Mab 1E4 binds specifically to the AA-sequences homologous to the bovine PrP₁₀₈₋₁₁₉ region and share the QWNKPS as core AA-sequence. Moreover an AA-sequence homologous to that of the epitope appears to be present in mouse and hamster PrP₂₁₇₋₂₂₂ region implying the AA Q109, K112 and S114 to be essential in the epitope for Mab 1E4 in the linear sequence QXXKXS.

### Figure 5

Summary of pepscan data of Mab 1E4 and other anti-PrP Mabs; P4, 2D6, 3F4, KG9, 6H4. Mab 1E4 was the only monoclonal antibody that reacted with all species tested.

### Figure 6

Multiple amino acid alignment of PrP of the most important animal species. Residues different from sheep PrP are boxed. Numbering is according to sheep PrP. Above the alignment a schematic representation is shown of the various regions or domains within prion proteins (PrPC). Structural element boundaries as determined by homology modeling (Swissmodel, Peitsch, 1996) of sheep PrP on basis of hamster and mouse structural data. Putative glycosylation sites (CHO) as well as octarepeat regions (R), alpha-helices (H), beta-sheets (S), and the disulphide bonds (S-S) are indicated separately (alpha-helices and beta-sheets only account for PrPC). The Proteinase-K cleavage site in PrPSc (conformational structure dependent) is indicated at region 82-94.

### Detailed Description

Here we describe the exposure of neo-epitopes by PrP^{Sc} upon cleavage by PK. These neo-epitopes can be used to identify a novel class of binding molecules for the detection of prions in tissues containing high concentrations of PrP^{C}. This class is exemplified by the isolation and characterisation of a new monoclonal antibody (1E4) that has a low affinity for the PrP^{C} and undigested PrP^{Sc} as compared to its affinity to PK-digested PrP^{Sc} the so-called PrP²⁷⁻³⁰ [the 27-30 kD fragment of PrP^{Sc} obtained after PK-digestion). The remarkable specificity of this antibody was found upon analysis of a series of antibodies generated against PrP-derived peptides mapped very close to the N-terminal end of PrP²⁷⁻³⁰. The new class of binding proteins will improve the detection of prion in several species including human, bovine, ovine, and murine, as is indicated by showing examples of application of this class of binding molecules in various test systems, including Western blotting, and immuno-histochemistry. Furthermore, we will show examples of using these molecules for strain typing within and between species. As an example of this approach we will describe the selection, isolation and characterisation of Mab 1E4.

### Examples

### Isolation of monoclonal antibody 1E4

Monoclonal antibody 1E4 was isolated from fusion of spleen cells of a Prnp^{0/0} mouse, immunised with the peptide GQWNKPSKPKTN# (corresponding to the bovine PrP AA sequence 108-119; #=amidated carboxy-terminus) coupled to KLH at its N-terminal end via a CG-AA linker. Sera from 6 out of 10 mice immunised with this KLH-coupled peptide reacted positive in scrapie/BSE dot-blot assay. Moreover sera from two mice contained high titers of antibodies as detected in anti-peptide ELISA and radioimmunoassay (RIA), detecting immobilised and fluid phase interaction, respectively. One of these mice was selected for fusion. Four days after a final boost with the KLH-coupled peptide, the spleen was collected, and spleen cells were isolated and fused with SP2/0-Ag14 myeloma cells. The fused cells were plated in 96-well plates at a density of 10⁵ cells/well. After 7 and 14 days of selection with HA (hypoxantine and azaserine), culture supernatants were screened for specific antibodies with the anti-peptide RIA, resulting in 23 positive wells. Limiting dilution of these clones revealed that only 10 clones were stable regarding IgG production as measured with the anti-peptide RIA. The cells from these 10 wells were subsequently subcloned three times by limiting dilution. Each subcloning step was monitored with the anti-peptide RIA, ultimately resulting in five stable clones. Culture supernatant of clone 1E4 was tested with Western blot demonstrating a strong binding reaction to BSE brain homogenates treated with PK, whereas virtually no binding was found to brain homogenates from healthy controls. This observation showed the unique binding characteristic on the 1E4 monoclonal antibody that was subsequently analysed in detail.

### Characterisation of monoclonal antibody 1E4

The binding properties of Mab 1E4 were compared with those of generally used Mab 6H4 (Prionics AG, Zürich, Switserland) using Western blot. BSE samples were treated with PK and subsequently three-fold serial dilutions of the samples were prepared. Mab 1E4 still yielded a positive reaction with a 243-fold dilution of the PK-treated sample, whereas the 81-fold dilution of the untreated sample was found negative (Figure 1). Using Mab 6H4 the 81-fold dilutions of both PK-treated and untreated BSE samples was found positive. Therefore this Western blot indicates that 1E4 has at least a nine-fold higher affinity for the PK-digested BSE sample as compared to untreated sample, assuming that 6H4 has a similar affinity for both types of antigens. This pivotal observation suggests that 1E4 has a higher affinity for the cleaved PrP²⁷⁻³⁰ than for the non-cleaved PrP^{Sc}. Moreover the Western blot also included a brain homogenate sample derived from a normal cow. Mab1E4 reacted only weakly with the latter sample, whereas 6H4 reacted very strongly with the sample suggesting that 1E4 has at least a nine-fold lower affinity for PrP^{C} than 6H4 has. So compared to 6H4, 1E4 has a lower affinity for PrP^{C} and PrP^{Sc}, but a higher affinity for PrP²⁷⁻³⁰.

These differences in affinity for PrP^{C}, PrP^{Sc}, and PrP²⁷⁻³⁰ were more striking using a spot-blot assay (Figure 2). Using Mab 1E4 the 270-fold dilution of the PK-treated BSE sample was found positive, whereas the 10-fold dilution of the untreated sample was found negative. Using Mab 6H4 however the 90-fold dilution of the PK-treated BSE sample was positive, whereas the 810-fold dilution of the untreated sample was found positive.

Furthermore Mab 1E4 did not react with brain homogenate derived from a normal cow, whereas Mab 6H4 reacted with the 2430-fold dilution of the sample. These findings confirm that 1E4 has a lower affinity for bovine PrP^{C} and PrP^{Sc}, but a higher affinity for PrP²⁷⁻³⁰ as compared to 6H4.

Mab 1E4 also reacted with prion from 301V-infected mice, scrapie-infected sheep and sCJD- and vCJD-infected human using Western blots (Figure 3). However the striking difference between the affinity for cleaved and non-cleaved PrP^{Sc} observed for BSE in cattle is not seen in these samples.

The broad species reactivity in the Western blot is also seen on the Pepscan analysis of Mab 1E4. As already indicated the antibody originated from a mouse immunised with KLH-cg-Bovine PrP₁₀₈₋₁₁₉: the AA-sequence was GQWNKPSKPKTN# and Pepscan analysis showed a linear epitope for Mab 1E4 present in this AA region in bovine, sheep, human, mouse and hamster with QWNKPS as core AA sequence (Figure 4). Furthermore a mimic of the epitope was found on mouse and hamster PrP₂₁₇₋₂₂₂ (AA sequence QYQKES) implying the AA Q109, K112 and S114 to be essential residues for antibody binding in the original epitope for Mab 1E4. Replacement analysis of PrP₂₂₀ AA K by R, as it is present by bovine, sheep and human, resulted in absence of binding activity to mouse and hamster in the Pepscan in this region.

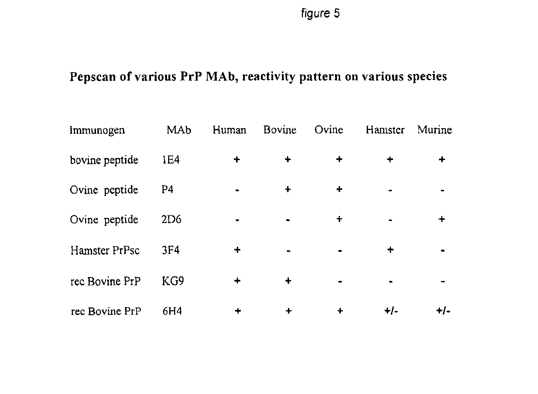

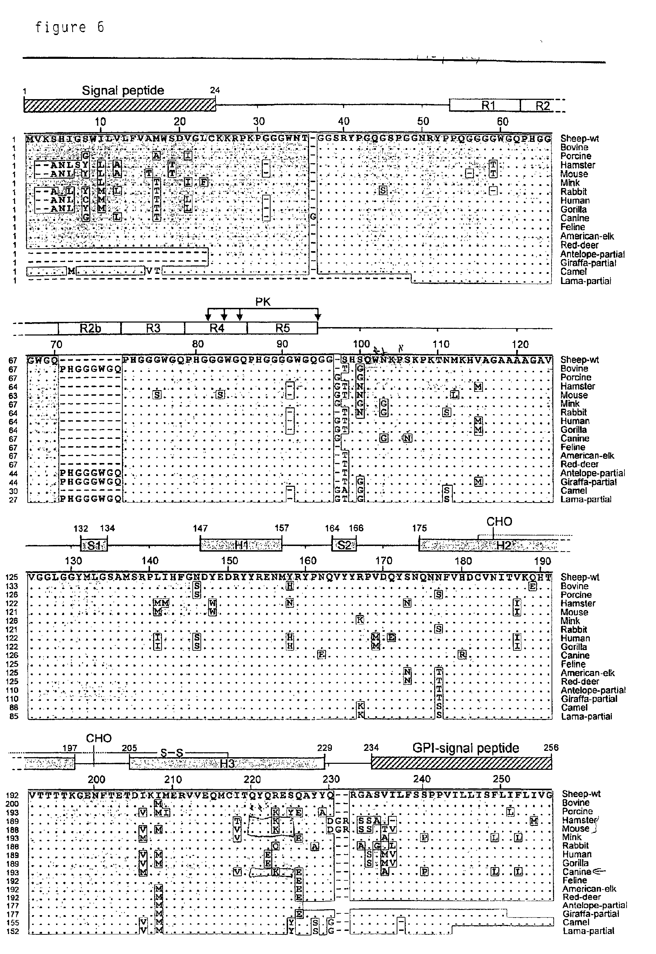

When we compared 1E4 with other PrP Mabs in Pepscan analysis, 1E4 appeared to be the only antibody having high affinities for a broad range of species (Figure 5). Therefore the antibody would be suitable as a general antibody for detection prions in general and for BSE in particular. The broad species reactivity of Mab 1E4 is also found when the antibody is used for immuno-histochemistry of sections from brainstem of BSE-infected cattle, brainstem of BSE- and scrapie-infected sheep, and total brain of BSE- and scrapie-infected mice.

To further characterise the immunoglobulin that is produced by clone 1E4, we RT-PCR-cloned the V_{H} region of the Ig expressed by clone 1E4. The amplified cDNA was sequenced and the hypervariable CDR3 region was identified. The CDR3 AA sequence of clone 1E4 is AGDNDAEDY.

### Novel applications using monoclonal antibody 1E4

The lower affinity for PrP^{C}, PrP^{Sc}, and higher affinity for PrP²⁷⁻³⁰ was particularly found for BSE-infected cattle, but not e.g. for scrapie-infected sheep. This property of Mab 1E4 might be exploited to facilitate discrimination between BSE and scrapie in sheep and human CJD cases. Hereby we assume that the position of PK-cleavage for prion in sheep with scrapie might differ from that in sheep with BSE. Thus, biochemical differences found between TSE strains (scrapie strains of hamster, scrapie of sheep, and BSE of cattle) have been reported. ^{6,13}

### Methods

### Monoclonal antibodies

P4, 2D6, 3F4, KG9, 6H4 (Prionics AG). ^{9, 10,11, 14, 15}

### Selection and synthesis of peptides

We selected the bovine PrP₁₀₈₋₁₁₉ AA sequence. The selected peptide cgGQWNKPSKPKTN, corresponding to the bovine PrP AA-sequence 108-119 plus the additional CG for flexibility and coupling, was synthesised from fmoc AA. Coupling of KLH to the N-terminal side of the peptide was done using a bifunctional linker MBS.

### Immunisation

The selected peptide was synthesised and coupled to KLH before immunising a group of five Prnp^{0/0} mice (Charles Weissmann [Zürich, Switserland]). Mice were immunised intraperitoneally using 30 µg of peptide suspended into Freunds Incomplete Adjuvans/PBS. Mice were boosted at 19, 59, and 136 days post immunisation (dpi).

### Screening of mice sera

Sera of the mice were collected at 73 dpi and analysed using various immuno-assays. Two different assays were used for detecting the peptide specific responses - anti-peptide ELISA and anti-peptide RIA - , whereas also assays were used to detect responses against PrP^{C} (PrP^{C} ELISA) and PrP^{Sc} (Scrapie/BSE dot-blot)

### Anti-peptide ELISA

ELISA plates were coated with BSA-coupled peptide and incubated with various dilutions of mice sera that were collected at -7 and 73 dpi. Bound antibodies were detected using a goat-anti-mouse-HRP conjugate and using TMB as a substrate.

### Anti-peptide RIA

Radiolabelled BSA-coupled peptide (¹²⁵I-peptide, iodogen method) was incubated with mice sera. Subsequently bound and free-labelled peptide was separated by addition of rat-anti-mouse-immunoglobulin coupled Sepharose, incubation and centrifugation. Gamma counter quantified radioactivity in the pellet.

### PrP^{C} ELISA

ELISA plates were coated with normal bovine or ovine crude brain homogenate and incubated with various dilutions of mice sera that were collected at -7 and 73 dpi. Then the bound antibodies were detected using a goat-anti-mouse-immunoglobulin-HRP conjugate and using TMB as a substrate.

### Scrapie/BSE dot-blot assay

Strips of PVDF membrane were spotted with PK-treated crude brain homogenate derived from normal sheep, scrapie-infected sheep, normal cattle and BSE-infected cattle. Samples were diluted three-fold and nine-fold and 3 µl spots were applied in duplicate. Subsequently the strips were incubated with various dilutions of mice sera that were collected 7 days before immunisation and 73 dpi. Bound antibodies were detected using a goat-anti-mouse-immunoglobulin-HRP conjugate, enhanced chemiluminescence as substrate, and visualised using NBT, BCIP as substrate.

### Fusion of spleen cells and preparation of Mab

Four days after the final booster, spleen cells of a selected mouse were fused with SP2/0-Ag14 myeloma cells in a 3:1 ratio and were plated in 96-well cluster plates (density 10⁵ cells/well). Hybridoma culture supernatants were screened for presence of specific antibodies by anti-peptide RIA and Scrapie/BSE dot-blot assay one and two weeks later.

### Selection and subcloning of Mab

Clones giving positive results in both anti-peptide RIA and scrapie/BSE dot-blot assays were subcloned three times by limiting dilution. Anti-peptide RIA was used to monitor production of specific antibodies during the cloning steps.

### Characterisation of Mab

After three limiting dilution subcloning cycles, the finally selected clones were characterised using Western blotting, spot-blotting, pepscan, immuno-histochemistry, and CDR3.

### Western blotting

Western blotting was carried out according to the Prionics Check kit specifications (Prionics AG). Briefly, proteins present in PK-treated or crude brain homogenates derived from normal sheep, scrapie infected sheep, normal cattle and BSE infected cattle were first separated on SDS-12.5% polyacrylamide gels and blotted on PVDF membranes. Subsequently the membranes were incubated with selected Mab supernatants. Bound antibodies were detected using a goat-anti-mouse-HRP conjugate, enhanced chemiluminescence as substrate, and visualised by exposure to ECL hyperfilm.

### Spot-blot

Protease-K-treated or crude brain homogenates derived from normal sheep, scrapie infected sheep, normal cattle and BSE infected cattle were diluted three-fold and nine-fold and 5 µl spots were applied to a nitrocellulose membrane. After drying and blocking the membranes were incubated with selected Mab supernatants. Bound antibodies were detected using a goat-anti-mouse-immunoglobulin HRP conjugate, enhanced chemiluminescence as substrate, and visualised by exposure to ECL hyperfilm.

### Pepscan

Solid phase synthesis of peptides on polyethylene pins and immunoscreening by ELISA were carried out according to established PEPSCAN™ procedures.^{18, 19} Complete sets were synthesised of overlapping 15-mer peptides in 3 µl polyethylene wells, covering the amino sequences of human, bovine, ovine, murine and hamster PrP and binding of antibodies to these sets was determined. ^{20, 21, 22, 28, 24}

### Immuno-histochemistry

Tissues were collected and embedded in paraffin and sections were cut, and stained with 1E4 (1:10 dilution).²⁵

### CDR3 determination

The V_{H} region of clone 1E4 was RT-PCR amplified using primers as described by Dattamajumdar et al. (1996).²⁶ The amplified PCR product was purified and used for sequence analysis.

### References

1 Prusiner SB, Prions (1998). PNAS 95, 13363-13383.
2 Wadsforth JDF et al., Tissue distribution of protease resistant prion protein in variant Creutzfeldt-Jakob disease using a highly sensitive immunoblotting assay (2001). Lancet 358, 171-180.
3 Schreuder BE et al., Preclinical test for prion diseases (1996). Nature 381, 563.
4 Naslavsky N, et al., Characterization of detergent-insoluble complexes containing the cellular prion protein and its scrapie isoform (1997). JBC 272, 6324-6331.
5 Wiessmann C, The ninth Datta Lecture. Molecular biology of transmissible spongiform encepholpathies (1996). FEBS Letters 389, 3-11.
6 Safar J et al., Eight prions strains have PrPSc molecules with different conformations (1998). Nature Medicine 4, 1157-1165.
7 Schmerr MJ and Jenny A, A diagnostic test for scrapie-infected sheep using a capillary electrophoresis immunoassay with fluorescent-labeled peptides (1998). Electrophoresis 19, 409-413.
8 Houston F et al.Transmission of BSE by blood transfusion in sheep (2000). Lancet 356, 999-1000
9 Kascsak RJ et al., Mouse polyclonal and monoclonal antibody to scrapie-associated fibrin proteins (1987). J Virol 61, 3688-3693.
10 Laffling AJ, et al. A monoclonal antibody that enables specific immunohistological detection of prion protein in bovine spongiform encephalopathy cases (2001). Neurosci Lett. 300, 99-102.
11 Korth C et al., Prion (PrPSc)-specific epitope defined by a monoclonal antibody (1997). Nature 390, 74-77.
12 MacGregor I, Prion protein and developments in its detection (2001). Transf Med 11, 3-14.
13 Madec JV et al., Sensitivity of the Western blot detection of protein PrPres in natural sheep scrapie (1998). J Virol Method 75, 169-177.
14 Harmeyer, S et al., Synthetic peptide vaccines yield monoclonal antibodies to cellular and pathological prion proteins of ruminants (1998). J Gen Virol 79, 937-945
15 Garssen GJ et al., Int. Patent application nr. PCT/NL00/00079 (2000)."Prion test".
16 Van Keulen LJ et al., Immunohistochemical detection and localization of prion protein in brain tissue of sheep with natural scrapie (1995). Vet-Pathol. 32, 299-308.
17 Garssen GJ et al., A rapid assay for BSE and scrapie. Int. Symp. "Characterization and Diagnosis of Prion Diseases in Animals and Man". Sept. 1999. Tubingen. Germany
18 Geysen HM et al., Use of peptide synthesis to probe viral antigens for epitopes to a resolution of a single amino acid (1984). PNAS 81, 3998-4002.
19 Piccardo P et al., An antibody raised against a conserved sequence of the prion protein recognizes pathological isoforms in human and animal prion diseases, including Creutzfeldt-Jakob disease and bovine spongiform encephalopathy (1998). Am J Pathol 152, 1415-1420.
20 Kretzschmar HA et al., Molecular cloning of a human prionprotein cDNA (1989). DNA 5, 315-324.
21 Goldmann W et al. Different forms of the bovine PrP gene have five or six copies of a short, G-C-rich element within the protein-coding exon (1991). J Gen Virol 72, 201-204.
22 Goldmann W et al., Two alleles of a neural protein gene linked to scrapie in sheep (1990). PNAS 87, 2476-2480.
23 Westaway D et al., Distinct prion proteins in short and long scrapie incubation period mice (1987). Cell 51,651-662.
24 Robakis NK et al., Isolation of a cDNA clone encoding the leader peptide of prion protein and expression of the homologous gene in various tissues (1986). PNAS 83, 6377-6381.
25 Van Keulen LJM et al., Immunohistochemical detection of prion protein in lymphoid tissues of sheep with natural scrapie (1996). J Clin Microbiol 34, 1228-1231.
26 Dattamajumbar AK et al., Rapid cloning of any rearranged mouse immunoglobulin variable genes (1996). Immunogenetics 43, 141-151.

## Claims

1. An antibody specifically reactive with an epitope which is exposed on a part of an aberrant conformer (PrP^{Sc}) of a prion protein after treatment of said conformer with a protease wherein said epitope is not or only partly exposed on a prion protein which has not been treated with a protease.

2. An antibody **characterized by** a higher affinity to an aberrant prion protein (PrP^{Sc} ) than to a non-aberrant prion protein (PrP^{c}).

3. An antibody according to claim 2 wherein said PrP^{Sc} has been treated with a protease.

4. An antibody according to anyone of claims 1 to 3 wherein said protease comprises protease K.

5. An antibody according to anyone of claims 1 to 4 wherein said epitope comprises a peptide with an amino acid sequence QXXKXS.

6. An antibody according to anyone of claims 1 to 5 with a specific reactivity to overlapping 15-mer oligo-peptides derived from the human, ovine, murine as well as hamster PrP amino-acid-sequences that correspond to the bovine PrP amino acid sequence at about amino acid position 108-119.

7. An antibody according to claim 6 wherein said bovine PrP amino acid sequence comprises GQWNKPSKPKTN.

8. An antibody according to claim 7 comprising a V_{H} region encoded by a nucleic acid comprising at least a CDR3 having a nucleic acid sequence encoding the amino acid sequence AGDNDAEDY.

9. A hybridoma cell capable of producing a monoclonal antibody of claim 7 or 8.

10. Hybridoma cell 1E4 having the deposit accession number CNCM 1-2906.

11. A method for determining the presence of a prion protein in a sample comprising using an antibody according to anyone of claims 1 to 8.

12. A method according to claim 11 for determining the presence of a prion protein in a biological sample via the detection of a complex between said protein and said antibody.

13. A diagnostic test kit for the determination of the presence of a prion protein in a biological sample comprising an antibody according to anyone of claims 1 to 8.

14. A method for obtaining an antibody according to anyone of claims 1 to 8 , comprising :
providing a set of antibody-producing cells by,
testing said cells for the presence of an antibody specifically reactive with PrP^{Sc}.
testing the ability of said antibody to react with higher affinity with PrP^{Sc} than with PrP^{C}.
selecting an antibody specifically reactive with protease-digested PrP^{Sc} comprising testing the ability of said antibody to react with higher affinity with protease-digested Prp^{Sc} material compared to non-digested PrP^{Sc} material

15. A method according to claim 14 further comprising selecting an antibody specifically reactive with PrP from multiple species

16. A method according to claim 15 further comprising testing said antibody for reactivity of said antibody with oligomerpeptides derived from the human, bovine, ovine, murine and hamster PrP.

17. A method according to anyone of claims 14-16 further comprising selecting an antibody with an additional reactivity with a peptide with an amino acid sequence QXXKXS comprising measuring the reactivity of said antibody with oligomerpeptides derived from murine as well as hamster PrP amino acid sequence that correspond to murine PrP amino acid sequence at about amino acid position 217-222 in a Pepscan analysis.


SEQUENCE LISTING

<110> Pepscan Systems B.V.
      Stichting Sanquin Bloedvoorziening

<120> Detection of prion disease

<130> P57843EP00

<140> 02077910.4
<141> 2002-07-17

<160> 21

<170> PatentIn Ver. 2.1

<210> 1
<211> 6
<212> PRT
<213> Mus

<220>
<221> SITE
<222> (1)..(6)
<223> /note="At least part of neo-epitope on the
      infectious form of prion protein, wherein X can be
      any amino acid"

<400> 1
Gln Xaa Xaa Lys Xaa Ser
  1               5


<210> 2
<211> 12
<212> PRT
<213> Bos

<220>
<221> SITE
<222> (1)..(12)
<223> /note="At least part of bovine PrP amino acid
      sequence"

<400> 2
Gly Gln Trp Asn Lys Pro Ser Lys Pro Lys Thr Asn
  1               5                  10


<210> 3
<211> 9
<212> PRT
<213> Artificial Sequence

<220>
<223> Description of Artificial Sequence: part of PrP
      binding peptide

<220>
<221> SITE
<222> (1)..(9)

<400> 3
Ala Gly Asp Asn Asp Ala Glu Asp Tyr
1               5

<210> 4
<211> 6
<212> PRT
<213> Bos

<220>
<221> SITE
<222> (1)..(6)
<223> /note="Part of bovine PrP 108-119 region"

<400> 4
Gln Trp Asn Lys Pro Ser
1               5

<210> 5
<211> 6
<212> PRT
<213> Mus

<220>
<221> SITE
<222> (1)..(6)
<223> /note="Mimic of Mab 1E4 epitope on PrP 217-222"

<400> 5
Gln Tyr Gln Lys Glu Ser
1               5

<210> 6
<211> 256
<212> PRT
<213> Ovis

<220>
<221> SITE
<222> (1)..(256)
<223> /note="Prion protein"

<400> 6
Met Val Lys Ser His Ile Gly Ser Trp Ile Leu Val Leu Phe Val Ala
1               5                   10                  15

Met Trp Ser Asp Val Gly Leu Cys Lys Lys Arg Pro Lys Pro Gly Gly
            20                  25                  30

Gly Trp Asn Thr Gly Gly Ser Arg Tyr Pro Gly Gln Gly Ser Pro Gly
        35                  40                  45

Gly Asn Arg Tyr Pro Pro Gln Gly Gly Gly Gly Trp Gly Gln Pro His
    50                  55                  60

Gly Gly Gly Trp Gly Gln Pro His Gly Gly Gly Trp Gly Gln Pro His
65                  70                  75                  80

Gly Gly Gly Trp Gly Gln Pro His Gly Gly Gly Gly Trp Gly Gln Gly
                85                  90                  95

Gly Ser His Ser Gln Trp Asn Lys Pro Ser Lys Pro Lys Thr Asn Met
            100                 105                 110

Lys His Val Ala Gly Ala Ala Ala Ala Gly Ala Val Val Gly Gly Leu
        115                 120                 125

Gly Gly Tyr Met Leu Gly Ser Ala Met Ser Arg Pro Leu Ile His Phe
    130                 135                 140

Gly Asn Asp Tyr Glu Asp Arg Tyr Tyr Arg Glu Asn Met Tyr Arg Tyr
145                 150                 155                 160

Pro Asn Gln Val Tyr Tyr Arg Pro Val Asp Gln Tyr Ser Asn Gln Asn
                165                 170                 175

Asn Phe Val His Asp Cys Val Asn Ile Thr Val Lys Gln His Thr Val
            180                 185                 190

Thr Thr Thr Thr Lys Gly Glu Asn Phe Thr Glu Thr Asp Ile Lys Ile
        195                 200                 205

Met Glu Arg Val Val Glu Gln Met Cys Ile Thr Gln Tyr Gln Arg Glu
    210                 215                 220

Ser Gln Ala Tyr Tyr Gln Arg Gly Ala Ser Val Ile Leu Phe Ser Ser
225                 230                 235                 240

Pro Pro Val Ile Leu Leu Ile Ser Phe Leu Ile Phe Leu Ile Val Gly
                245                 250                 255


<210> 7
<211> 264
<212> PRT
<213> Bos

<220>
<221> SITE
<222> (1)..(264)
<223> /note="Prion protein"

<400> 7
Met Val Lys Ser His Ile Gly Ser Trp Ile Leu Val Leu Phe Val Ala
1               5                   10                  15

Met Trp Ser Asp Val Gly Leu Cys Lys Lys Arg Pro Lys Pro Gly Gly
            20                  25                  30

Gly Trp Asn Thr Gly Gly Ser Arg Tyr Pro Gly Gln Gly Ser Pro Gly
        35                  40                  45

Gly Asn Arg Tyr Pro Pro Gln Gly Gly Gly Gly Trp Gly Gln Pro His
    50                  55                  60

Gly Gly Gly Trp Gly Gln Pro His Gly Gly Gly Trp Gly Gln Pro His
65                  70                  75                  80

Gly Gly Gly Trp Gly Gln Pro His Gly Gly Gly Trp Gly Gln Pro His
                85                  90                  95

Gly Gly Gly Gly Trp Gly Gln Gly Gly Thr His Gly Gln Trp Asn Lys
            100                 105                 110

Pro Ser Lys Pro Lys Thr Asn Met Lys His Val Ala Gly Ala Ala Ala
        115                 120                 125

Ala Gly Ala Val Val Gly Gly Leu Gly Gly Tyr Met Leu Gly Ser Ala
    130                 135                 140

Met Ser Arg Pro Leu Ile His Phe Gly Ser Asp Tyr Glu Asp Arg Tyr
145                 150                 155                 160

Tyr Arg Glu Asn Met His Arg Tyr Pro Asn Gln Val Tyr Tyr Arg Pro
                165                 170                 175

Val Asp Gln Tyr Ser Asn Gln Asn Asn Phe Val His Asp Cys Val Asn
            180                 185                 190

Ile Thr Val Lys Glu His Thr Val Thr Thr Thr Thr Lys Gly Glu Asn
        195                 200                 205

Phe Thr Glu Thr Asp Ile Lys Met Met Glu Arg Val Val Glu Gln Met
    210                 215                 220

Cys Ile Thr Gln Tyr Gln Arg Glu Ser Gln Ala Tyr Tyr Gln Arg Gly
225                 230                 235                 240

Ala Ser Val Ile Leu Phe Ser Ser Pro Pro Val Ile Leu Leu Ile Ser
                245                 250                 255

Phe Leu Ile Phe Leu Ile Val Gly
            260


<210> 8
<211> 257
<212> PRT
<213> Porcinus

<220>
<221> SITE
<222> (1)..(257)
<223> /note="Prion protein"

<400> 8
Met Val Lys Ser His Ile Gly Gly Trp Ile Leu Val Leu Phe Val Ala
1               5                   10                  15

Ala Trp Ser Asp Ile Gly Leu Cys Lys Lys Arg Pro Lys Pro Gly Gly
            20                  25                  30

Gly Trp Asn Thr Gly Gly Ser Arg Tyr Pro Gly Gln Gly Ser Pro Gly
        35                  40                  45

Gly Asn Arg Tyr Pro Pro Gln Gly Gly Gly Gly Trp Gly Gln Pro His
    50                  55                  60

Gly Gly Gly Trp Gly Gln Pro His Gly Gly Gly Trp Gly Gln Pro His

65                  70                  75                  80

Gly Gly Gly Trp Gly Gln Pro His Gly Gly Gly Gly Trp Gly Gln Gly
                85                  90                  95

Gly Gly Ser His Gly Gln Trp Asn Lys Pro Ser Lys Pro Lys Thr Asn
            100                 105                 110

Met Lys His Val Ala Gly Ala Ala Ala Ala Gly Ala Val Val Gly Gly
        115                 120                 125

Leu Gly Gly Tyr Met Leu Gly Ser Ala Met Ser Arg Pro Leu Ile His
    130                 135                 140

Phe Gly Ser Asp Tyr Glu Asp Arg Tyr Tyr Arg Glu Asn Met Tyr Arg
145                 150                 155                 160

Tyr Pro Asn Gln Val Tyr Tyr Arg Pro Val Asp Gln Tyr Ser Asn Gln
                165                 170                 175

Asn Ser Phe Val His Asp Cys Val Asn Ile Thr Val Lys Gln His Thr
            180                 185                 190

Val Thr Thr Thr Thr Lys Gly Glu Asn Phe Thr Glu Thr Asp Val Lys
        195                 200                 205

Met Ile Glu Arg Val Val Glu Gln Met Cys Ile Thr Gln Tyr Gln Lys
    210                 215                 220

Glu Tyr Glu Ala Tyr Ala Gln Arg Gly Ala Ser Val Ile Leu Phe Ser
225                 230                 235                 240

Ser Pro Pro Val Ile Leu Leu Ile Ser Phe Leu Leu Phe Leu Ile Val
                245                 250                 255

Gly

<210> 9
<211> 254
<212> PRT
<213> Hamustro

<220>
<221> SITE
<222> (1)..(254)
<223> /note="Prion protein"

<400> 9
Met Ala Asn Leu Ser Tyr Trp Leu Leu Ala Leu Phe Val Ala Met Trp
1               5                   10                  15

Thr Asp Val Gly Leu Cys Lys Lys Arg Pro Lys Pro Gly Gly Trp Asn
            20                  25                  30

Thr Gly Gly Ser Arg Tyr Pro Gly Gln Gly Ser Pro Gly Gly Asn Arg
        35                  40                  45

Tyr Pro Pro Gln Gly Gly Gly Thr Trp Gly Gln Pro His Gly Gly Gly
    50                  55                  60

Trp Gly Gln Pro His Gly Gly Gly Trp Gly Gln Pro His Gly Gly Gly
65                  70                  75                  80

Trp Gly Gln Pro His Gly Gly Gly Trp Gly Gln Gly Gly Gly Thr His
                85                  90                  95

Asn Gln Trp Asn Lys Pro Ser Lys Pro Lys Thr Asn Met Lys His Met
            100                 105                 110

Ala Gly Ala Ala Ala Ala Gly Ala Val Val Gly Gly Leu Gly Gly Tyr
        115                 120                 125

Met Leu Gly Ser Ala Met Ser Arg Pro Met Met His Phe Gly Asn Asp
    130                 135                 140

Trp Glu Asp Arg Tyr Tyr Arg Glu Asn Met Asn Arg Tyr Pro Asn Gln
145                 150                 155                 160

Val Tyr Tyr Arg Pro Val Asp Gln Tyr Asn Asn Gln Asn Asn Phe Val
                165                 170                 175

His Asp Cys Val Asn Ile Thr Ile Lys Gln His Thr Val Thr Thr Thr
            180                 185                 190

Thr Lys Gly Glu Asn Phe Thr Glu Thr Asp Ile Lys Ile Met Glu Arg
        195                 200                 205

Val Val Glu Gln Met Cys Thr Thr Gln Tyr Gln Lys Glu Ser Gln Ala
    210                 215                 220

Tyr Tyr Asp Gly Arg Arg Ser Ser Ala Val Leu Phe Ser Ser Pro Pro
225                 230                 235                 240

Val Ile Leu Leu Ile Ser Phe Leu Ile Phe Leu Met Val Gly
                245                 250


<210> 10
<211> 254
<212> PRT
<213> Mus

<220>
<221> SITE
<222> (1)..(254)
<223> /note="Prion protein"

<400> 10
Met Ala Asn Leu Gly Tyr Trp Leu Leu Ala Leu Phe Val Thr Met Trp
1               5                   10                  15

Thr Asp Val Gly Leu Cys Lys Lys Arg Pro Lys Pro Gly Gly Trp Asn
            20                  25                  30

Thr Gly Gly Ser Arg Tyr Pro Gly Gln Gly Ser Pro Gly Gly Asn Arg
        35                  40                  45

Tyr Pro Pro Gln Gly Gly Thr Trp Gly Gln Pro His Gly Gly Gly Trp
    50                  55                  60

Gly Gln Pro His Gly Gly Ser Trp Gly Gln Pro His Gly Gly Ser Trp
65                  70                  75                  80

Gly Gln Pro His Gly Gly Gly Trp Gly Gln Gly Gly Gly Thr His Asn
                85                  90                  95

Gln Trp Asn Lys Pro Ser Lys Pro Lys Thr Asn Leu Lys His Val Ala
            100                 105                 110

Gly Ala Ala Ala Ala Gly Ala Val Val Gly Gly Leu Gly Gly Tyr Met
        115                 120                 125

Leu Gly Ser Ala Met Ser Arg Pro Met Ile His Phe Gly Asn Asp Trp
    130                 135                 140

Glu Asp Arg Tyr Tyr Arg Glu Asn Met Tyr Arg Tyr Pro Asn Gln Val
145                 150                 155                 160

Tyr Tyr Arg Pro Val Asp Gln Tyr Ser Asn Gln Asn Asn Phe Val His
                165                 170                 175

Asp Cys Val Asn Ile Thr Ile Lys Gln His Thr Val Thr Thr Thr Thr
            180                 185                 190

Lys Gly Glu Asn Phe Thr Glu Thr Asp Val Lys Met Met Glu Arg Val
        195                 200                 205

Val Glu Gln Met Cys Val Thr Gln Tyr Gln Lys Glu Ser Gln Ala Tyr
    210                 215                 220

Tyr Asp Gly Arg Arg Ser Ser Ser Thr Val Leu Phe Ser Ser Pro Pro
225                 230                 235                 240

Val Ile Leu Leu Ile Ser Phe Leu Ile Phe Leu Ile Val Gly
                245                 250

<210> 11
<211> 257
<212> PRT
<213> Mustela

<220>
<221> SITE
<222> (1)..(257)
<223> /note="Prion protein"

<400> 11
Met Val Lys Ser His Ile Gly Ser Trp Leu Leu Val Leu Phe Val Ala
1               5                   10                  15

Thr Trp Ser Asp Ile Gly Phe Cys Lys Lys Arg Pro Lys Pro Gly Gly
            20                  25                  30

Gly Trp Asn Thr Gly Gly Ser Arg Tyr Pro Gly Gln Gly Ser Pro Gly
        35                  40                  45

Gly Asn Arg Tyr Pro Pro Gln Gly Gly Gly Gly Trp Gly Gln Pro His
    50                  55                  60

Gly Gly Gly Trp Gly Gln Pro His Gly Gly Gly Trp Gly Gln Pro His

65                  70                  75                  80

Gly Gly Gly Trp Gly Gln Pro His Gly Gly Gly Gly Trp Gly Gln Gly
                85                  90                  95

Gly Gly Ser His Gly Gln Trp Gly Lys Pro Ser Lys Pro Lys Thr Asn
            100                 105                 110

Met Lys His Val Ala Gly Ala Ala Ala Ala Gly Ala Val Val Gly Gly
        115                 120                 125

Leu Gly Gly Tyr Met Leu Gly Ser Ala Met Ser Arg Pro Leu Ile His
    130                 135                 140

Phe Gly Asn Asp Tyr Glu Asp Arg Tyr Tyr Arg Glu Asn Met Tyr Arg
145                 150                 155                 160

Tyr Pro Asn Gln Val Tyr Tyr Lys Pro Val Asp Gln Tyr Ser Asn Gln
                165                 170                 175

Asn Asn Phe Val His Asp Cys Val Asn Ile Thr Val Lys Gln His Thr
            180                 185                 190

Val Thr Thr Thr Thr Lys Gly Glu Asn Phe Thr Glu Thr Asp Met Lys
        195                 200                 205

Ile Met Glu Arg Val Val Glu Gln Met Cys Val Thr Gln Tyr Gln Arg
    210                 215                 220

Glu Ser Glu Ala Tyr Tyr Gln Arg Gly Ala Ser Ala Ile Leu Phe Ser
225                 230                 235                 240

Pro Pro Pro Val Ile Leu Leu Ile Ser Leu Leu Ile Leu Leu Ile Val
                245                 250                 255

Gly

<210> 12
<211> 252
<212> PRT
<213> Oryctolagus

<220>
<221> SITE
<222> (1)..(252)
<223> /note="Prion protein"

<400> 12
Met Ala His Leu Gly Tyr Trp Met Leu Leu Leu Phe Val Ala Thr Trp
1               5                   10                  15

Ser Asp Val Gly Leu Cys Lys Lys Arg Pro Lys Pro Gly Gly Gly Trp
            20                  25                  30

Asn Thr Gly Gly Ser Arg Tyr Pro Gly Gln Ser Ser Pro Gly Gly Asn
        35                  40                  45

Arg Tyr Pro Pro Gln Gly Gly Gly Trp Gly Gln Pro His Gly Gly Gly
    50                  55                  60

Trp Gly Gln Pro His Gly Gly Gly Trp Gly Gln Pro His Gly Gly Gly
65                  70                  75                  80

Trp Gly Gln Pro His Gly Gly Gly Trp Gly Gln Gly Gly Thr His Asn
                85                  90                  95

Gln Trp Gly Lys Pro Ser Lys Pro Lys Thr Ser Met Lys His Val Ala
            100                 105                 110

Gly Ala Ala Ala Ala Gly Ala Val Val Gly Gly Leu Gly Gly Tyr Met
        115                 120                 125

Leu Gly Ser Ala Met Ser Arg Pro Leu Ile His Phe Gly Asn Asp Tyr
    130                 135                 140

Glu Asp Arg Tyr Tyr Arg Glu Asn Met Tyr Arg Tyr Pro Asn Gln Val
145                 150                 155                 160

Tyr Tyr Arg Pro Val Asp Gln Tyr Ser Asn Gln Asn Ser Phe Val His
                165                 170                 175

Asp Cys Val Asn Ile Thr Val Lys Gln His Thr Val Thr Thr Thr Thr
            180                 185                 190

Lys Gly Glu Asn Phe Thr Glu Thr Asp Ile Lys Ile Met Glu Arg Val
        195                 200                 205

Val Glu Gln Met Cys Ile Thr Gln Tyr Gln Gln Glu Ser Gln Ala Ala
    210                 215                 220

Tyr Gln Arg Ala Ala Gly Val Leu Leu Phe Ser Ser Pro Pro Val Ile
225                 230                 235                 240

Leu Leu Ile Ser Phe Leu Ile Phe Leu Ile Val Gly
                245                 250


<210> 13
<211> 253
<212> PRT
<213> Homo sapiens

<220>
<221> SITE
<222> (1)..(253)
<223> /note="Prion protein"

<400> 13


<210> 14
<211> 253
<212> PRT
<213> Gorilla

<220>
<221> SITE
<222> (1)..(253)
<223> /note="Prion protein"

<400> 14
Met Ala Asn Leu Gly Tyr Trp Met Leu Val Leu Phe Val Ala Thr Trp
  1               5                  10                  15

Ser Asp Leu Gly Leu Cys Lys Lys Arg Pro Lys Pro Gly Gly Trp Asn
             20                  25                  30

Thr Gly Gly Ser Arg Tyr Pro Gly Gln Gly Ser Pro Gly Gly Asn Arg
         35                  40                  45

Tyr Pro Pro Gln Gly Gly Gly Gly Trp Gly Gln Pro His Gly Gly Gly
     50                  55                  60

Trp Gly Gln Pro His Gly Gly Gly Trp Gly Gln Pro His Gly Gly Gly

65                  70                  75                  80

Trp Gly Gln Pro His Gly Gly Gly Trp Gly Gln Gly Gly Gly Thr His
                85                  90                  95

Ser Gln Trp Asn Lys Pro Ser Lys Pro Lys Thr Asn Met Lys His Met
            100                 105                 110

Ala Gly Ala Ala Ala Ala Gly Ala Val Val Gly Gly Leu Gly Gly Tyr
        115                 120                 125

Met Leu Gly Ser Ala Met Ser Arg Pro Ile Ile His Phe Gly Ser Asp
    130                 135                 140

Tyr Glu Asp Arg Tyr Tyr Arg Glu Asn Met His Arg Tyr Pro Asn Gln
145                 150                 155                 160

Val Tyr Tyr Arg Pro Met Asp Gln Tyr Ser Asn Gln Asn Asn Phe Val
                165                 170                 175

His Asp Cys Val Asn Ile Thr Ile Lys Gln His Thr Val Thr Thr Thr
            180                 185                 190

Thr Lys Gly Glu Asn Phe Thr Glu Thr Asp Val Lys Met Met Glu Arg
        195                 200                 205

Val Val Glu Gln Met Cys Ile Thr Gln Tyr Glu Arg Glu Ser Gln Ala
    210                 215                 220

Tyr Tyr Gln Arg Gly Ser Ser Met Val Leu Phe Ser Ser Pro Pro Val
225                 230                 235                 240

Ile Leu Leu Ile Ser Phe Leu Ile Phe Leu Ile Val Gly
                245                 250


<210> 15
<211> 257
<212> PRT
<213> Caninus

<220>
<221> SITE
<222> (1)..(257)
<223> /note="Prion protein"

<400> 15
Met Val Lys Ser His Ile Gly Gly Trp Ile Leu Leu Leu Phe Val Ala
1               5                   10                  15

Thr Trp Ser Asp Val Gly Leu Cys Lys Lys Arg Pro Lys Pro Gly Gly
            20                  25                  30

Trp Asn Thr Gly Gly Gly Ser Arg Tyr Pro Gly Gln Gly Ser Pro Gly
        35                  40                  45

Gly Asn Arg Tyr Pro Pro Gln Gly Gly Gly Gly Trp Gly Gln Pro His
    50                  55                  60

Gly Gly Gly Trp Gly Gln Pro His Gly Gly Gly Trp Gly Gln Pro His
65                  70                  75                  80

Gly Gly Gly Trp Gly Gln Pro His Gly Gly Gly Gly Trp Gly Gln Gly
                85                  90                  95

Gly Gly Ser His Ser Gln Trp Gly Lys Pro Asn Lys Pro Lys Thr Asn
            100                 105                 110

Met Lys His Val Ala Gly Ala Ala Ala Ala Gly Ala Val Val Gly Gly
        115                 120                 125

Leu Gly Gly Tyr Met Leu Gly Ser Ala Met Ser Arg Pro Leu Ile His
    130                 135                 140

Phe Gly Asn Asp Tyr Glu Asp Arg Tyr Tyr Arg Glu Asn Met Tyr Arg
145                 150                 155                 160

Tyr Pro Glu Gln Val Tyr Tyr Arg Pro Val Asp Gln Tyr Ser Asn Gln
                165                 170                 175

Asn Asn Phe Val Arg Asp Cys Val Asn Ile Thr Val Lys Gln His Thr
            180                 185                 190

Val Thr Thr Thr Thr Lys Gly Glu Asn Phe Thr Glu Thr Asp Met Lys
        195                 200                 205

Ile Met Glu Arg Val Val Glu Gln Met Cys Val Thr Gln Tyr Gln Lys
    210                 215                 220

Glu Ser Glu Ala Tyr Tyr Gln Arg Gly Ala Ser Ala Ile Leu Phe Ser
225                 230                 235                 240

Pro Pro Pro Val Ile Leu Leu Ile Ser Leu Leu Ile Leu Leu Ile Val
                245                 250                 255

Gly

<210> 16
<211> 256
<212> PRT
<213> Felinus

<220>
<221> SITE
<222> (1)..(256)
<223> /note="Prion protein"

<400> 16
Met Val Lys Ser His Ile Gly Ser Trp Ile Leu Val Leu Phe Val Ala
  1               5                  10                  15

Met Trp Ser Asp Val Gly Leu Cys Lys Lys Arg Pro Lys Pro Gly Gly
             20                  25                  30

Gly Trp Asn Thr Gly Gly Ser Arg Tyr Pro Gly Gln Gly Ser Pro Gly
         35                  40                  45

Gly Asn Arg Tyr Pro Pro Gln Gly Gly Gly Gly Trp Gly Gln Pro His
     50                  55                  60

Gly Gly Gly Trp Gly Gln Pro His Gly Gly Gly Trp Gly Gln Pro His
65                  70                  75                  80

Gly Gly Gly Trp Gly Gln Pro His Gly Gly Gly Gly Trp Gly Gln Gly
                85                  90                  95

Gly Ser His Ser Gln Trp Asn Lys Pro Ser Lys Pro Lys Thr Asn Met
            100                 105                 110

Lys His Val Ala Gly Ala Ala Ala Ala Gly Ala Val Val Gly Gly Leu
        115                 120                 125

Gly Gly Tyr Met Leu Gly Ser Ala Met Ser Arg Pro Leu Ile His Phe
    130                 135                 140

Gly Asn Asp Tyr Glu Asp Arg Tyr Tyr Arg Glu Asn Met Tyr Arg Tyr
145                 150                 155                 160

Pro Asn Gln Val Tyr Tyr Arg Pro Val Asp Gln Tyr Ser Asn Gln Asn
                165                 170                 175

Asn Phe Val His Asp Cys Val Asn Ile Thr Val Lys Gln His Thr Val
            180                 185                 190

Thr Thr Thr Thr Lys Gly Glu Asn Phe Thr Glu Thr Asp Ile Lys Ile
        195                 200                 205

Met Glu Arg Val Val Glu Gln Met Cys Ile Thr Gln Tyr Gln Arg Glu
    210                 215                 220

Ser Glu Ala Tyr Tyr Gln Arg Gly Ala Ser Val Ile Leu Phe Ser Ser
225                 230                 235                 240

Pro Pro Val Ile Leu Leu Ile Ser Phe Leu Ile Phe Leu Ile Val Gly
                245                 250                 255


<210> 17
<211> 256
<212> PRT
<213> Cervus elaphus

<220>
<221> SITE
<222> (1)..(256)
<223> /note="Prion protein"

<400> 17
Met Val Lys Ser His Ile Gly Ser Trp Ile Leu Val Leu Phe Val Ala
1               5                   10                  15

Met Trp Ser Asp Val Gly Leu Cys Lys Lys Arg Pro Lys Pro Gly Gly
            20                  25                  30

Gly Trp Asn Thr Gly Gly Ser Arg Tyr Pro Gly Gln Gly Ser Pro Gly
        35                  40                  45

Gly Asn Arg Tyr Pro Pro Gln Gly Gly Gly Gly Trp Gly Gln Pro His
    50                  55                  60

Gly Gly Gly Trp Gly Gln Pro His Gly Gly Gly Trp Gly Gln Pro His

65                  70                  75                  80

Gly Gly Gly Trp Gly Gln Pro His Gly Gly Gly Gly Trp Gly Gln Gly
                85                  90                  95

Gly Thr His Ser Gln Trp Asn Lys Pro Ser Lys Pro Lys Thr Asn Met
            100                 105                 110

Lys His Val Ala Gly Ala Ala Ala Ala Gly Ala Val Val Gly Gly Leu
        115                 120                 125

Gly Gly Tyr Met Leu Gly Ser Ala Met Ser Arg Pro Leu Ile His Phe
    130                 135                 140

Gly Asn Asp Tyr Glu Asp Arg Tyr Tyr Arg Glu Asn Met Tyr Arg Tyr
145                 150                 155                 160

Pro Asn Gln Val Tyr Tyr Arg Pro Val Asp Gln Tyr Asn Asn Gln Asn
                165                 170                 175

Thr Phe Val His Asp Cys Val Asn Ile Thr Val Lys Gln His Thr Val
            180                 185                 190

Thr Thr Thr Thr Lys Gly Glu Asn Phe Thr Glu Thr Asp Ile Lys Met
        195                 200                 205

Met Glu Arg Val Val Glu Gln Met Cys Ile Thr Gln Tyr Gln Arg Glu
    210                 215                 220

Ser Glu Ala Tyr Tyr Gln Arg Gly Ala Ser Val Ile Leu Phe Ser Ser
225                 230                 235                 240

Pro Pro Val Ile Leu Leu Ile Ser Phe Leu Ile Phe Leu Ile Val Gly
                245                 250                 255


<210> 18
<211> 211
<212> PRT
<213> Antalopus

<220>
<221> SITE
<222> (1)..(211)
<223> /note="Part of prion protein"

<400> 18
Cys Lys Lys Arg Pro Lys Pro Gly Gly Gly Trp Asn Thr Gly Gly Ser
1               5                   10                  15

Arg Tyr Pro Gly Gln Gly Ser Pro Gly Gly Asn Arg Tyr Pro Pro Gln
            20                  25                  30

Gly Gly Gly Gly Trp Gly Gln Pro His Gly Gly Gly Trp Gly Gln Pro
        35                  40                  45

His Gly Gly Gly Trp Gly Gln Pro His Gly Gly Gly Trp Gly Gln Pro
    50                  55                  60

His Gly Gly Gly Trp Gly Gln Pro His Gly Gly Gly Gly Trp Gly Gln
65                  70                  75                  80

Gly Gly Thr His Ser Gln Trp Asn Lys Pro Ser Lys Pro Lys Thr Asn
                85                  90                  95

Met Lys His Val Ala Gly Ala Ala Ala Ala Gly Ala Val Val Gly Gly
            100                 105                 110

Leu Gly Gly Tyr Met Leu Gly Ser Ala Met Ser Arg Pro Leu Ile His
        115                 120                 125

Phe Gly Asn Asp Tyr Glu Asp Arg Tyr Tyr Arg Glu Asn Met Tyr Arg
    130                 135                 140

Tyr Pro Asn Gln Val Tyr Tyr Arg Pro Val Asp Gln Tyr Ser Asn Gln
145                 150                 155                 160

Asn Thr Phe Val His Asp Cys Val Asn Ile Thr Val Lys Gln His Thr
                165                 170                 175

Val Thr Thr Thr Thr Lys Gly Glu Asn Phe Thr Glu Thr Asp Ile Lys
            180                 185                 190

Met Met Glu Arg Val Val Glu Gln Met Cys Ile Thr Gln Tyr Gln Arg
        195                 200                 205

Glu Ser Gln
    210


<210> 19
<211> 235
<212> PRT
<213> Giraffa

<220>
<221> SITE
<222> (1)..(235)
<223> /note="Part of prion protein"

<400> 19
Cys Lys Lys Arg Pro Lys Pro Gly Gly Gly Trp Asn Thr Gly Gly Ser
1               5                   10                  15

Arg Tyr Pro Gly Gln Gly Ser Pro Gly Gly Asn Arg Tyr Pro Pro Gln
            20                  25                  30

Gly Gly Gly Gly Trp Gly Gln Pro His Gly Gly Gly Trp Gly Gln Pro
        35                  40                  45

His Gly Gly Gly Trp Gly Gln Pro His Gly Gly Gly Trp Gly Gln Pro
    50                  55                  60

His Gly Gly Gly Trp Gly Gln Pro His Gly Gly Gly Gly Trp Gly Gln
65                  70                  75                  80

Gly Gly Thr His Gly Gln Trp Asn Lys Pro Ser Lys Pro Lys Thr Asn
                85                  90                  95

Met Lys His Met Ala Gly Ala Ala Ala Ala Gly Ala Val Val Gly Gly
            100                 105                 110

Leu Gly Gly Tyr Met Leu Gly Ser Ala Met Ser Arg Pro Leu Ile His
        115                 120                 125

Phe Gly Asn Asp Tyr Glu Asp Arg Tyr Tyr Arg Glu Asn Met Tyr Arg
    130                 135                 140

Tyr Pro Asn Gln Val Tyr Tyr Arg Pro Val Asp Gln Tyr Ser Asn Gln
145                 150                 155                 160

Asn Thr Phe Val His Asp Cys Val Asn Ile Thr Val Lys Gln His Thr
                165                 170                 175

Val Thr Thr Thr Thr Lys Gly Glu Asn Phe Thr Glu Thr Asp Ile Lys
            180                 185                 190

Met Met Glu Arg Val Val Glu Gln Met Cys Ile Thr Gln Tyr Gln Arg
        195                 200                 205

Glu Ser Glu Ala Tyr Tyr Gln Arg Gly Ala Ser Val Ile Leu Phe Ser
    210                 215                 220

Ser Pro Pro Val Ile Leu Leu Ile Ser Phe Leu
225                 230                 235


<210> 20
<211> 255
<212> PRT
<213> Camelus

<220>
<221> SITE
<222> (1)..(255)
<223> /note="Prion protein"

<400> 20
Met Val Lys Ser His Met Gly Ser Trp Ile Leu Val Leu Phe Val Val
1               5                   10                  15

Thr Trp Ser Asp Val Gly Leu Cys Lys Lys Arg Pro Lys Pro Gly Gly
            20                  25                  30

Gly Trp Asn Thr Gly Gly Ser Arg Tyr Pro Gly Gln Gly Ser Pro Gly
        35                  40                  45

Gly Asn Arg Tyr Pro Pro Gln Gly Gly Gly Gly Trp Gly Gln Pro His
    50                  55                  60

Gly Gly Gly Trp Gly Gln Pro His Gly Gly Gly Trp Gly Gln Pro His
65                  70                  75                  80

Gly Gly Gly Trp Gly Gln Pro His Gly Gly Gly Trp Gly Gln Gly Gly
                85                  90                  95

Gly Ala His Gly Gln Trp Asn Lys Pro Ser Lys Pro Lys Thr Ser Met
            100                 105                 110

Lys His Val Ala Gly Ala Ala Ala Ala Gly Ala Val Val Gly Gly Leu
        115                 120                 125

Gly Gly Tyr Met Leu Gly Ser Ala Met Ser Arg Pro Leu Ile His Phe

130                 135                 140

Gly Asn Asp Tyr Glu Asp Arg Tyr Tyr Arg Glu Asn Met Tyr Arg Tyr
145                 150                 155                 160

Pro Asn Gln Val Tyr Tyr Lys Pro Val Asp Gln Tyr Ser Asn Gln Asn
                165                 170                 175

Ser Phe Val His Asp Cys Val Asn Ile Thr Val Lys Gln His Thr Val
            180                 185                 190

Thr Thr Thr Thr Lys Gly Glu Asn Phe Thr Glu Thr Asp Val Lys Met
        195                 200                 205

Met Glu Arg Val Val Glu Gln Met Cys Ile Thr Gln Tyr Gln Arg Glu
    210                 215                 220

Tyr Gln Ala Ser Tyr Gly Arg Gly Ala Ser Val Ile Phe Ser Ser Pro
225                 230                 235                 240

Pro Val Ile Leu Leu Ile Ser Phe Leu Ile Phe Leu Ile Val Gly
                245                 250                 255


<210> 21
<211> 202
<212> PRT
<213> Lama

<220>
<221> SITE
<222> (1)..(202)
<223> /note="Part of prion protein"

<400> 21
Gly Asn Arg Tyr Pro Pro Gln Gly Gly Gly Gly Trp Gly Gln Pro His
1               5                   10                  15

Gly Gly Gly Trp Gly Gln Pro His Gly Gly Gly Trp Gly Gln Pro His
            20                  25                  30

Gly Gly Gly Trp Gly Gln Pro His Gly Gly Gly Trp Gly Gln Pro His
        35                  40                  45

Gly Gly Gly Trp Gly Gln Gly Gly Gly Thr His Gly Gln Trp Asn Lys
    50                  55                  60

Pro Ser Lys Pro Lys Thr Ser Met Lys His Val Ala Gly Ala Ala Ala
65                  70                  75                  80

Ala Gly Ala Val Val Gly Gly Leu Gly Gly Tyr Met Leu Gly Ser Ala
                85                  90                  95

Met Ser Arg Pro Leu Ile His Phe Gly Asn Asp Tyr Glu Asp Arg Tyr
            100                 105                 110

Tyr Arg Glu Asn Met Tyr Arg Tyr Pro Asn Gln Val Tyr Tyr Lys Pro
        115                 120                 125

Val Asp Gln Tyr Ser Asn Gln Asn Ser Phe Val His Asp Cys Val Asn
    130                 135                 140

Ile Thr Val Lys Gln His Thr Val Thr Thr Thr Thr Lys Gly Glu Asn
145                 150                 155                 160

Phe Thr Glu Thr Asp Val Lys Met Met Glu Arg Val Val Glu Gln Met
                165                 170                 175

Cys Ile Thr Gln Tyr Gln Arg Glu Tyr Gln Ala Ser Tyr Gly Arg Gly
            180                 185                 190

Ala Ser Val Ile Phe Ser Ser Pro Pro Val
        195                 200